(19) Europäisches Patentamt
European Patent Office
Office européen des brevets

(11) Numéro de publication : **0 033 959**
**B1**

(12) # FASCICULE DE BREVET EUROPÉEN

(45) Date de publication du fascicule du brevet : **11.05.83**

(21) Numéro de dépôt : **81100860.6**

(22) Date de dépôt : **06.02.81**

(51) Int. Cl.³ : **C 07 D307/94**, A 23 L 1/226// C07C35/08, C07C29/42, C07C49/603

(54) **Composé spirannique insaturé, son utilisation dans les parfums et les arômes et procédé pour sa préparation.**

(30) Priorité : **08.02.80 CH 1013/80**

(43) Date de publication de la demande : **19.08.81 Bulletin 81/33**

(45) Mention de la délivrance du brevet : **11.05.83 Bulletin 83/19**

(84) Etats contractants désignés : **CH DE FR GB LI NL**

(56) Documents cités :
**FR A 2 319 640**
**JOURNAL OF ORGANOMETALLIC CHEMISTRY, vol. 168, 1979, LAUSANNE (CH) J. G. DUBOUDIN et al. : « Réactifs de Grignard vinyliques gamma fonctionnels III* Addition à quelques dérivés carbonyles », pages 233-240**

(73) Titulaire : **FIRMENICH SA**
**Case postale 239**
**CH-1211 Genève 8 (CH)**

(72) Inventeur : **Fankhauser, Peter**
**23 avenue des Grandes-Communes**
**CH-1213 ONEX/GE (CH)**

(74) Mandataire : **Schmied-Kowarzik, Volker, Dr., et al**
**Patentanwälte Dr. V. Schmied-Kowarzik Dipl.-Ing. G. Dannenberg Dr. P. Weinhold Dr. D. Gudel Dipl.-Ing. S. Schubert Siegfriedstrasse 8**
**D-8000 München 40 (DE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 033 959 B1

Composé spirannique insaturé, son utilisation dans les parfums et les arômes et procédé pour sa préparation

L'invention se rapporte à un composé nouveau utile à l'industrie des parfums et arômes. Elle a plus précisément pour objet le 6-éthyl-2,10,10-triméthyl-1-oxa-spiro [4.5] déca-3,6-diène, de formule

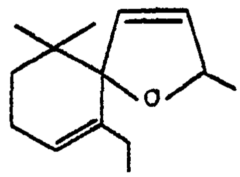

(I)

et son utilisation à titre d'ingrédient parfumant ou aromatisant. Elle a également pour objet une composition parfumante, respectivement aromatisante, contenant ledit composé de formule (I) à titre d'ingrédient organoleptiquement actif.

L'invention a plus particulièrement pour objet l'utilisation dudit composé de formule (I) pour conférer une note olfactive de type cassis exempte de tonalité soufrée à des parfums, bases ou compositions parfumantes ou produits parfumés.

L'invention a finalement pour objet un procédé pour la préparation dudit composé de formule (I).

Parmi les notes olfactives qui connaissent un certain renouveau en parfumerie moderne, on peut citer la note fruitée de type cassis. Fait étonnant, l'industrie des parfums ne dispose actuellement que de fort peu de substances odorantes capables de reproduire efficacement cette note olfactive très particulière. Celle-ci se retrouve notamment dans l'absolu de bourgeons de cassis (Ribes Nigrum), une matière d'origine naturelle au demeurant fort rare et fort chère, accompagnée cependant d'odeurs annexes parfois difficiles à masquer. Cette note fruitée de type cassis se retrouve également, à des degrés divers, dans plusieurs composés chimiques synthétiques, tels le 2,6,9,10,10-pentaméthyl-1-oxa-spiro [4.5] déca-3,6-diène et le 2,6,9,10-tétraméthyl-1-oxa-spiro [4.5] déca-3,6-diène, respectivement de formules

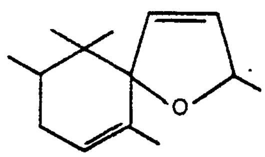

et

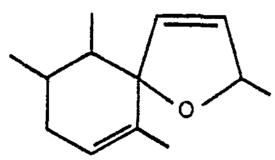

dont l'odeur est caractérisée par une note fraîche, verte et naturelle, rappelant par certains côtés l'odeur des feuilles de menthe sauvage, de sauge sclarée et du cassis, ainsi que par une note fruitée rappelant plus ou moins celle des agrumes, le pamplemousse en particulier [voir par exemple DE-A-2 634 077 = FR-A-2 319 640 et DE-A-2 749 511, respectivement]. Elle se retrouve également dans le menthone-thiol-8, de formule

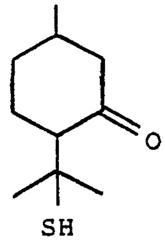

[voir par exemple DE-C-2 008 254], mais accompagnée dans ce cas-ci d'une tonalité soufrée. Ce composé est en outre d'un emploi limité vu sa relative instabilité dans des milieux agressifs tels que savons, shampooings ou détergents par exemple.

Contrairement à ce que l'état de la technique précité permettait de conclure, il a été trouvé de façon surprenante qu'il était désormais possible, par l'emploi du composé de formule (I) objet de l'invention, de combler efficacement cette lacune, à savoir la reproduction d'une note olfactive de type cassis, exempte de tonalité soufrée. Ceci peut se faire en outre d'autant plus avantageusement que ledit composé s'est révélé olfactivement stable dans des produits tels que savons, détergents ou produits d'entretien par exemple.

Dans le domaine des parfums, le composé (I) se distingue par une note olfactive originale de type cassis, exempte de tonalité soufrée et rappelant de ce fait le côté fruité de l'absolu de bourgeons de cassis. Il peut être par conséquent avantageusement ajouté à de nombreuses compositions parfumantes, telles des compositions de type fruité, frais, menthé, fleuri, rosé, chypré ou boisé par exemple, au sein desquelles il développe cette note de type cassis exempte de tonalité soufrée qui le caractérise. Il contribue ainsi à donner harmonie et originalité à de telles compositions parfumantes.

Le composé (I) peut être tout aussi avantageusement utilisé pour la préparation de produits parfumés tels que savons, poudres à lessive, shampooings, lotions capillaires, produits cosmétiques ou produits d'entretien par exemple.

Des effets olfactifs tels que définis ci-dessus peuvent être obtenus par l'emploi dudit composé à titre d'ingrédient odoriférant unique, par exemple sous forme de solution dans les solvants conventionnels tels l'alcool éthylique, le phtalate de diéthyle, le dipropylène-glycol ou le citrate d'éthyle, ou encore sous forme de mélange avec les ingrédients odoriférants usuels en parfumerie, dans ce cas sous forme de cœurs ou bases pour parfums par exemple.

L'effet olfactif obtenu par l'emploi dudit composé (I) dépend notamment de la concentration et, par exemple, de la nature des coingrédients de la base ou composition à laquelle on l'a incorporé. Lors de la préparation de compositions parfumantes par exemple, on peut obtenir des effets olfactifs intéressants par l'emploi de quantités de l'ordre de 0,05 % (poids) déjà. Les effets les plus caractéristiques s'obtiennent de préférence par l'emploi de quantités comprises entre 0,1 et 5 % environ du poids de la composition considérée, des quantités de l'ordre de 20 % ou plus pouvant être également envisagées, notamment lorsque des effets plus particuliers sont recherchés.

Dans le domaine des arômes, le composé (I) se caractérise par un goût fruité-boisé caractéristique, rappelant celui du cassis. Ledit composé peut être de ce fait avantageusement utilisé pour la préparation d'arômes artificiels, tels des arômes de fruits comme le cassis, la framboise ou autres baies rouges par exemple. Ledit composé développe en effet au sein de tels arômes une note fraîche, fruitée et boisée qui, dans la plupart des cas, renforce le côté naturel de tels arômes.

Le composé (I) peut être également utilisé pour l'aromatisation d'aliments, de boissons, de préparations pharmaceutiques ou du tabac. Des effets gustatifs tels que ceux décrits plus haut peuvent s'obtenir par l'emploi de quantités comprises entre 0,01 et 10 ppm environ, par rapport au poids du produit aromatisé. On utilise de préférence des quantités comprises entre 0,1 et 2, selon les cas 5 ppm. Des quantités supérieures à 10 ppm peuvent être également envisagées, notamment lorsque des effets plus particuliers sont recherchés.

Conformément au procédé de l'invention (méthode A), le composé (I) peut être obtenu après réaction du but-1-yne-3-ol avec la 2-éthyl-6,6-diméthyl-cyclohexanone, en présence d'une base forte. A titre de base forte, on peut avantageusement utiliser une base minérale ou organique telle que l'hydroxyde de potassium, le butyl-lithium, le tert-butylate de potassium ou l'amidure de sodium ou lithium par exemple. Ladite réaction peut s'effectuer en présence ou non d'un solvant organique inerte, dans un tel cas de préférence en préférence d'un éther, un hydrocarbure ou un mélange d'hydrocarbures aliphatiques ou aromatiques. Ladite réaction s'effectue en outre à une température le plus généralement comprise entre environ 25 et 55 °C, de préférence voisine de 35 °C.

Après les traitements usuels d'extraction, on isole un composé acétylénique nouveau, de formule

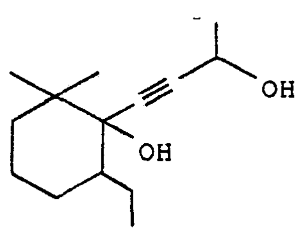

(III)

destiné à être ensuite soumis à un traitement acide. Ledit traitement acide s'effectue au moyen d'un acide fort, minéral ou organique, de préférence en milieu organique aqueux. A titre de solvant organique, on peut avantageusement utiliser les solvants définis pour l'étape réactionnelle précédente et, à titre d'acide fort, l'acide formique, phosphorique, sulfurique, polyphosphorique, benzènesulfonique ou p-toluène-sulfonique par exemple, ou encore une terre de diatomées acide. Ledit traitement s'effectue de préférence à une température comprise entre environ 50 et 100 °C, cette dernière coïncidant le plus généralement avec la température d'ébullition du mélange de réaction.

Selon une autre mise en œuvre du procédé de l'invention (méthode A), le composé (I) peut être également préparé à partir de 2-éthyl-6,6-diméthyl-cyclohexanone, après réaction avec un dérivé organo-métallique de formule (II) tel que défini précédemment, hydrolyse subséquente et traitement acide. Les conditions réactionnelles appliquées sont analogues à celles décrites ci-dessus.

Selon le procédé de l'invention (méthode B), le composé (I) peut être également obtenu à partir de 2-éthyl-6,6-diméthyl-cyclohex-2-ène-1-one, après réaction de celle-ci avec un composé organo-métallique

de formule (II) tel que défini précédemment, hydrolyse subséquente, hydrogénation du produit résultant et finalement traitement acide du produit d'hydrogénation.

La réaction de la 2-éthyl-6,6-diméthyl-cyclohex-2-ène-1-one avec le composé de formule (II) s'effectue selon les conditions usuelles en chimie organique, par exemple dans les conditions d'une réaction de Grignard. Après hydrolyse, on isole un composé acétylénique nouveau, de formule

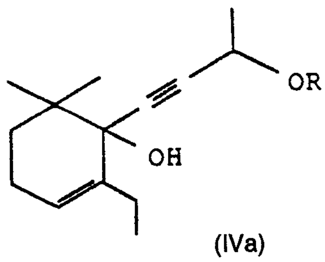
(IVa)

ou

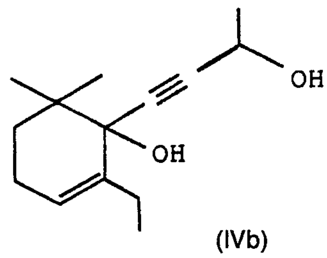
(IVb)

(R≠Mg-halogène) que l'on soumet ensuite à hydrogénation. Ladite hydrogénation s'effectue en présence d'un catalyseur métallique, le nickel de Raney par exemple, ou encore en présence d'un catalyseur partiellement inactivé, le catalyseur dit de Lindlar par exemple. On obtient ainsi un composé à chaîne latérale insaturée, de formule

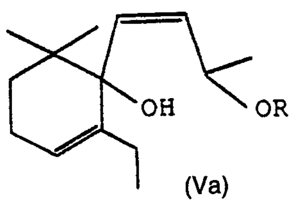
(Va)

ou

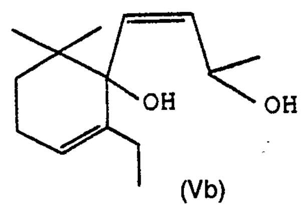
(Vb)

(R≠Mg-halogène) finalement soumis à un traitement acide aux conditions décrites précédemment concernant le composé de formule (III).

Tel qu'obtenu de la synthèse, le composé (I) peut se présenter sous forme d'un mélange stéréoisomérique tel que défini ci-dessous

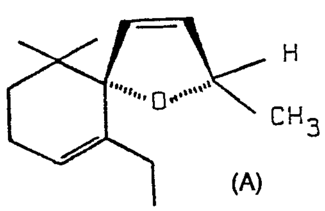
(A)

+

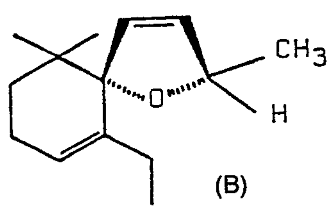
(B)

ou encore sous forme d'un seul desdits stéréoisomères. La formule (I) telle que définie au sein de la présente invention sert donc à désigner indifféremment l'un desdits stéréoisomères à titre individuel ou tout mélange desdits stéréoisomères.

Pour des raisons d'ordre pratique et économique, on utilisera ledit composé selon l'invention tel que directement issu de la synthèse. Les exemples ci-après illustrent l'invention de façon plus détaillée (température en degrés centigrades).

Exemple 1

Préparation du 6-éthyl-2,10,10-triméthyl-1-oxa-spiro [4.5] déca-3,6-diène

Méthode A

i) Dans un réacteur muni d'un système d'agitation et d'un réfrigérant, on a premièrement introduit 448 g (8 mole) de KOH et 1 100 ml de di-isopropyl-éther. 154 g (2,2 mole) de but-1-yne-3-ol ont ensuite été ajoutés au mélange ci-dessus (température : 25-30° ; temps d'introduction : 30 minutes), suivis de 308 g (2,0 mole) de 2-éthyl-6,6-diméthyl-cyclohexanone ajoutés goutte à goutte, en 30 minutes. Après

avoir agité le mélange réactionnel durant 17 h à 35°, on y a progressivement ajouté, sous refroidissement, 500 ml d'eau. Après décantation, la phase organique a été lavée avec $H_2SO_4$ à 5 % dans l'eau (200 ml), séchée sur $Na_2SO_4$ et finalement concentrée. Par distillation du résidu, on a premièrement recueilli 46 g de 2-éthyl-6,6-diméthyl-cyclohexanone non réagie, suivis de 313 g de 2-éthyl-6,6-diméthyl-1-hydroxy-1-(3-hydroxy-but-1-yne-1-yl)-cyclohexane (rendement 78 % sur produit de départ transformé). Un échantillon pour analyse a été purifié par recristallisation dans le cyclohexane, F. 136°.

IR : 3 600, 2 390, 1 210, 1 035, 920 $cm^{-1}$

RMN : 1,00 (3H, t, J = 7 Hz) ; 1,00 et 1,09 (6H, 2s) ; 1,48 (3H, d, J = 7 Hz) ; 1,0-1,9 (9H, m) ; 1,97 (2H, larges) ; 4,59 (1H, q, J = 7 Hz) δ ppm

SM : m/e = 206(7), 188(63), 173(76), 162(26), 147(100), 131(47), 117(54), 105(67), 91(79), 82(83), 69(58), 55(79), 43(88), 41(94), 29(62).

ii) 100 g (0,45 mole) du composé obtenu ci-dessus, en mélange avec 175 g d'acide phosphorique à 86 % et 200 ml de tétrahydrofuranne, ont été chauffés durant 20 h à reflux. Après refroidissement, le mélange de réaction a été additionné de 100 ml d'éther de pétrole (50/70) et lavé ensuite avec successivement $H_2O$ puis $NaHCO_3$ à 5 % dans l'eau. Après séchage sur $Na_2SO_4$, concentration et distillation fractionnée du résidu, on a recueilli 31 g (rendement 34 %) du produit désiré, Eb. 90°/3 × $10^2$ Pascals.

Selon l'analyse par chromatographie en phase gazeuse (colonne de silicone — UCON ; 120-180°), le produit ainsi obtenu se présente sous la forme d'un mélange stéréoisomérique 1 : 1.

IR (liq.) : 1 450, 1 375, 1 360, 1 355, 1 345, 1 110, 1 075, 1 045, 1 030, 1 000, 970, 940, 910, 860, 830, 800, 745, 710 $cm^{-1}$

RMN : 0,85 et 0,87 (2 × 1,5H, 2s) ; 0,93 (3H, s) ; 0,98 (3H, t, J = 7 Hz) ; 1,30 (3H, d, J = 6 Hz) ; 1,4-1,7 (2H, m) ; 1,7-2,2 (4H, m) ; 4,9 (1H, m) ; 5,38 et 5,49 (2 × 0,5H, 2m) ; 5,58 (1H, dxd, $J_1$ = 6 Hz), $J_2$ = 2 Hz) ; 5,77 et 5,84 (2 × 0,5H, d × d, $J_1$ = 6 Hz, $J_2$ = 1 Hz) δ ppm

SM : m/e = 150(100), 135(28), 121(52), 107(6), 91(7), 79(8), 67(2), 55(4), 43(18), 29(3).

La 2-éthyl-6,6-diméthyl-cyclohexanone utilisée ci-dessus comme produit de départ a été obtenue comme suit : 600 g (3,95 mole) de 2-éthyl-6,6-diméthyl-cyclohex-2-ène-1-one — préparée à partir de n-propyl-isopropyl-cétone et acroléine selon DE-A-2 547 223 — ont été hydrogénés à pression et température ambiantes en présence de 15 g de palladium à 5 % sur charbon. Après absorption d'environ 93 l de $H_2$ et filtration du catalyseur, on a recueilli 601 g (rendement 99 %) du produit désiré. Un échantillon pour analyse a été caractérisé comme indiqué ci-après :

Eb. 76°/ 16 × $10^2$ Pascals

IR : 1 705, 1 450, 1 380, 1 365, 1 130, 1 035, 990 $cm^{-1}$

SM : $M^+$ = 154(20) ; m/e = 126(6), 82(100), 69(31), 56(38), 55(33), 41(32), 27(9).

Méthode B

i) à un mélange de 14 g (0,042 mole) d'une solution de bromure d'éthylmagnésium à 40 % dans l'éther et 7,5 ml d'éther anhydre, on a ajouté goutte à goutte 6,0 g (0,042 mole) de 3-(3-oxa-pent-2-yloxy)-but-1-yne (addition sous atmosphère d'azote — température de réaction : 20° — période : 30 min). Après avoir été chauffé durant 1 h à reflux, le mélange réactionnel a été refroidi à 20° puis additionné de 3,8 g (0,025 mole) de 2-éthyl-6,6-diméthyl-cyclohex-2-ène-1-one, l'agitation du mélange étant poursuivie durant 1 h à température ambiante. Après refroidissement à 0-5°, on a successivement introduit 5 ml de $H_2O$ et 15 ml de $H_2SO_4$ à 5 % dans l'eau. Après extraction à l'éther, lavage, séchage de la phase organique sur $Na_2SO_4$ et évaporation, on a isolé 7,7 g de résidu brut. Après distillation (150°/13 Pascals), on a recueilli 4,4 g (rendement 59 %) d'un composé de formule

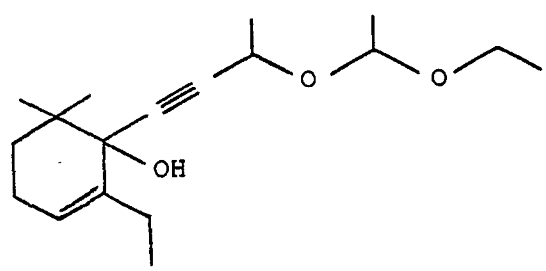

Ce dernier a été identifié comme suit :

RMN : signaux à 1,0-2,6 ; 3,3-4,0 ; 4,2-5,1 ; 5,3-5,6 ppm

SM : m/e = 232(17), 217(11), 204(26), 192(39), 175(15), 163(26), 148(85), 133(96), 120(47), 105(80), 91(40), 73(59), 55(34), 45(100), 43(91), 29(40).

ii) 2,9 g (0,01 mole) du composé obtenu ci-dessus dans 20 ml d'acétate d'éthyle ont été hydrogénés à

pression et température ambiantes en présence de 0,5 g de catalyseur de Lindlar (consommation de $H_2$ : 280 ml). Après filtration et évaporation, on a isolé 2,9 g (rendement 99 %) d'un composé de formule

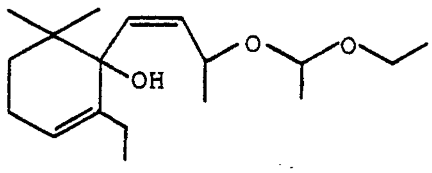

Un échantillon pour analyse a été purifié par distillation (110°/7 Pascals).

IR : 3 610, 3 450, 1 700, 1 655, 1 460, 1 380, 1 150-1 020 $cm^{-1}$

RMN : 0,8-1,7 (20H) ; 1,9-2,3 (4H, m) ; 2,8 et 2,9 (1H, 2s) ; 3,2-3,9 (2H, m) ; 4,6-5,7 (5H, m) δ ppm

SM : $M^+$ = 206(13) ; m/e = 194(13), 177(9), 163(5), 150(58), 135(25), 121(48), 107(22), 93(16), 79(12), 73(46), 55(28), 45(67), 43(100), 31(68).

iii) 1,0 g (3,4 mmole) du composé obtenu ci-dessus dans 5 ml d'éther de pétrole (80/100) ont été chauffés durant 5 h à reflux, sous atmosphère d'azote, en présence de 4 g de $H_2SO_4$ à 30 % dans l'eau. Après lavage de la phase organique avec une solution de $NaHCO_3$, séchage sur $Na_2SO_4$, évaporation et distillation, on a isolé 0,6 g (rendement 86 %) de 6-éthyl-2,10,10-triméthyl-1-oxa-spiro [4.5] déca-3,6-diène.

Le produit ainsi préparé s'est révélé identique à celui obtenu selon la méthode A décrite précédemment.

## Exemple 2

On a préparé une composition parfumante de base comme indiqué ci-après :

| Ingrédients | Parties en poids |
|---|---|
| IRALIA®1) | 160 |
| Acétate de vétivéryle | 100 |
| Salicylate de benzyle | 100 |
| Santalol | 80 |
| Alcool phényléthylique | 80 |
| CYCLOSIA®1) | 80 |
| Musc cétone | 70 |
| Essence de bergamote | 60 |
| Absolu de jasmin du Maroc | 60 |
| Absolu de rose de mai | 40 |
| Teinture de civette synth. | 40 |
| Teinture de musc Tonkin synth. | 30 |
| EXALTOLIDE®1) | 30 |
| Eugénol extra | 30 |
| Essence de rose bulgare | 15 |
| Aldéhyde undécylénique à 10 %* | 15 |
| Total | 990 |

* dans le phtalate diéthylique
1) origine : FIRMENICH SA, Genève-Suisse

En ajoutant à 99 g de la base ci-dessus 1 g de 6-éthyl-2,10,10-trimétyl-1-oxa-spiro [4.5] déca-3,6-diène, on a conféré à ladite base une note olfactive de type fruité-frais relativement montante, harmonieuse, rappelant l'odeur des bourgeons de cassis. Ladite base en est devenue d'autant plus riche et plaisante.

## Exemple 3

On a premièrement préparé une base parfumante comme indiqué ci-après :

| Ingrédients | Parties en poids |
|---|---|
| Citronellol | 200 |
| Benzoate de benzyle | 100 |
| Aldéhyde α-amyl-cinnamique | 80 |

| | |
|---|---|
| Hydroxy-citronellol | 80 |
| Essence d'ylang-ylang | 60 |
| Alcool phényléthylique | 60 |
| HEDIONE®1) | 40 |
| Propionate de phénoxyéthyle | 30 |
| α-Damascone à 1 %*1) | 20 |
| Linalol | 20 |
| Géranylacétone | 10 |
| Total | 700 |

* phtalate diéthyle
1) origine : FIRMENICH SA, Genève-Suisse

La base ainsi préparée se caractérise par une odeur générique qualifiée de fleurie.

En ajoutant à 700 g de la base ci-dessus 300 g de 6-éthyl-2,10,10-triméthyl-1-oxa-spiro [4.5] déca-3,6-diène, on lui confère une note de type fruité-cassis très caractéristique. La composition de type « cassis » ainsi obtenue possède l'avantage de se combiner harmonieusement à un grand nombre de compositions parfumantes.

Exemple 4

100 mg de 6-éthyl-2,10,10-triméthyl-1-oxa-spiro [4.5] déca-3,6-diène ont été incorporés à 100 g d'une pâte à savon commerciale, olfactivement neutre. La pâte ainsi parfumée a ensuite été façonnée selon les techniques usuelles en savons de toilette et ceux-ci soumis à une évaluation olfactive. Il a été constaté que lesdits savons dégageaient une odeur plaisante, de type fruité-cassis.

Exemple 5

On a procédé à l'aromatisation d'un sirop de cassis — préparé par dilution d'une partie de sirop concentré commercial dans 9 parties d'eau — en y ajoutant le 6-éthyl-2,10,10-triméthyl-1-oxa-spiro [4.5] déca-3,6-diène à raison de 5 ppm.

Après comparaison avec un sirop non aromatisé (témoin), on a constaté que le sirop aromatisé possédait un goût plus arrondi, plus naturel, et qu'il développait une note fruitée et boisée caractéristique.

**Revendications**

1. Composé de formule

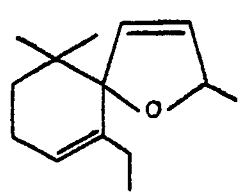

(I)

2. Utilisation du composé de formule (I) telle que définie à la revendication 1, à titre d'ingrédient parfumant ou aromatisant.

3. Utilisation du composé de formule (I) telle que définie à la revendication 1 pour conférer une note olfactive de type cassis exempte de tonalité soufrée à des parfums, bases ou compositions parfumantes ou produits parfumés.

4. Composition parfumante ou aromatisante, caractérisée en ce qu'elle contient le composé de formule (I) telle que définie à la revendication 1 à titre d'ingrédient organoleptiquement actif.

5. Procédé pour la préparation du composé de formule (I) telle que définie à la revendication 1, caractérisé en ce que :

A. on fait réagir la 2-éthyl-6,6-diméthyl-cyclohexanone avec le but-1-yne-3-ol en présence d'une base forte ou avec un composé organo-métallique de formule

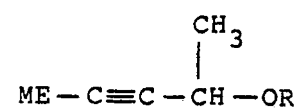

(II)

dans laquelle le symbole ME représente un métal alcalin ou un radical Mg-halogène et R représente un groupe trialkyl-silyle, tétrahydropyranyle, tert-butyle, 3-oxapent-2-yle, 3-oxa-but-2-yle ou un radical Mg-halogène, on hydrolyse et finalement traite au moyen d'un agent acide le produit de réaction obtenu ; ou

B. on fait réagir la 2-éthyl-6,6-diméthyl-cyclohex-2-ène-1-one avec un dérivé métallique de formule (II) telle que définie ci-dessus, on hydrolyse le produit de réaction obtenu, on hydrogène ensuite le produit résultant de ladite hydrolyse et finalement traite au moyen d'un agent acide ledit produit d'hydrogénation.

**Claims**

1. Compound of formula

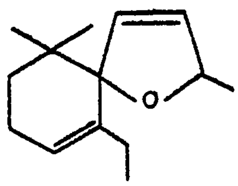

(I)

2. Utilization of the compound of formula (I) as defined in claim 1, as perfuming or flavoring ingredient.

3. Utilization of the compound of formula (I) as defined in claim 1 to impart to perfumes, perfuming bases or compositions, or perfumed products a black-currant type note free from sulfury tonality.

4. Perfuming or flavouring composition, characterized in that it contains the compound of formula (I), as defined in claim 1, as organoleptically active ingredient.

5. Process for the preparation of the compound of formula (I) as defined in claim 1, characterized in that :

A. 2-ethyl-6,6-dimethyl-cyclohexanone is reacted with but-1-yn-3-ol in the presence of a strong base or with an organo-metallic compound of formula

$$\mathrm{ME-C{\equiv}C-\underset{}{\overset{\displaystyle CH_3}{\overset{|}{CH}}}-OR} \qquad \text{(II)}$$

wherein symbol ME represents an alkali metal or a Mg-halogen radical and R represents a trialkyl-silyl, a tetrahydropyranyl, a tert-butyl, a 3-oxa-pent-2-yl, a 3-oxa-but-2-yl or a Mg-halogen radical, and the obtained reaction product is hydrolyzed and eventually treated with an acidic agent ; or

B. 2-ethyl-6,6-dimethyl-cyclohex-2-en-1-one is reacted with a metal derivative of formula (II) as defined above, the obtained reaction product is hydrolyzed, the resulting product is then hydrogenated and the said hydrogenated product is treated eventually with an acidic agent.

**Ansprüche**

1. Verbindung der Formel

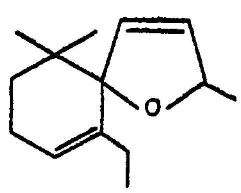

(I)

2. Verwendung der Verbindung der Formel (I) gemäss Anspruch 1, als Parfüm- oder Aromabestandteil.

3. Verwendung der Verbindung der Formel (I) gemäss Anspruch 1, um Parfümbasen, Parfümkompositionen oder parfümierten Produkten eine von schwefelartigem Charakter freie schwarze Johannisbeer Note zu verleihen.

4. Parfüm- oder Aromakomposition, dadurch gekennzeichnet, dass sie als organoleptisch aktiven Bestandteil die Verbindung der Formel (I) gemäss Anspruch 1 enthält.

5. Verfahren zur Herstellung der Verbindung der Formel (I) gemäss Anspruch 1, dadurch gekennzeichnet, dass man

A. 2-Aethyl-6,6-dimethyl-cyclohexanon mit But-1-in-3-ol in Anwesenheit einer starken Base oder mit einer organo-metallischen Verbindung der Formel

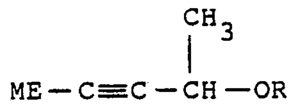

(II)

worin das Symbol ME ein Alkali-metall oder ein Mg-Halogen-Rest ist und R Trialkyl-silyl, Tetrahydropyranyl, tert-Butyl, 3-Oxa-pent-2-enyl, 3-Oxa-but-2-yl oder Mg-Halogen bedeutet, umsetzt und das so erhaltene Produkt hydrolysiert und dann mit einem sauren Reagenz behandelt ; oder

B. 2-Aethyl-6,6-dimethyl-cyclohex-2-en-1-on mit einem Metall-Derivat der oben angegebenen Formel (II) umsetzt, das Reaktionsprodukt hydrolisiert, das erhaltene Produkte hydriert und schließlich das Hydrierungsprodukt mit einem sauren Reagenz behandelt.